# EUROPEAN PATENT APPLICATION

(11) **EP 3 991 630 A1**
(43) Date of publication of application: **04.05.2022**
(21) Application number: 20855881.7
(22) Date of filing: 18.03.2020
(51) Int. Cl.: A61B 1/00

(54) **ENDOSCOPE CONNECTION APPARATUS AND ENDOSCOPE SYSTEM**

(30) Priority: 23.08.2019 CN 201921386670 U; 23.08.2019 CN 201921384591 U; 23.08.2019 CN 201921380586 U; 23.08.2019 CN 201910784437; 23.08.2019 CN 201921384593 U; 23.08.2019 CN 201910787484; 23.08.2019 CN 201921381523 U
(71) Applicant: Chongqing Jinshan Science & Technology (Group) Co., Ltd., Chongqing 401120 (CN)
(72) Inventor: SUN, Yu, Chongqing, 401120 (CN); DENG, Anpeng, Chongqing, 401120 (CN); WANG, Liao, Chongqing, 401120 (CN); CHEN, Kui, Chongqing, 401120 (CN); ZHOU, Jian, Chongqing, 401120 (CN); LI, Shixing, Chongqing, 401120 (CN); LI, Jianxia, Chongqing, 401120 (CN); YUAN, Moukun, Chongqing, 401120 (CN)
(74) Representative: Ipside
(86) International application number: PCT/CN2020/080004
(87) International publication number: WO 2021/036233

(57) **Abstract**

The present invention provides an endoscope connection device and an endoscope system, belonging to the technical field of endoscopes. A connection locking structure includes a connector I and a connector II, wherein the connector I is connected with the connector II through a magnetic connection assembly; the connection device includes the connection locking structure; and the endoscope system includes the connection device. Contact power supply is adopted in the present invention, no risk of electromagnetic leakage exists, a small structural size is achieved, and connector miniaturization is facilitated. Two optical signal transmission paths are adopted in the present invention to transmit image information and control information, respectively, to achieve separation of signal transmission from power supply and high interference resistance of the signal.

## Description

### Technical Field

The present invention belongs to the technical field of endoscopes and relates to an endoscope connection device and an endoscope system.

### Background of the Invention

An endoscope system widely used in the medical field includes an endoscope body, a processor and a cold light source, and is configured in two modes: a first mode in which the processor and the cold light source are arranged separately, and the endoscope body is separately connected with the processor and the cold light source; and a second mode in which the processor and the cold light source form an endoscope host, and the endoscope body is connected with the endoscope host.

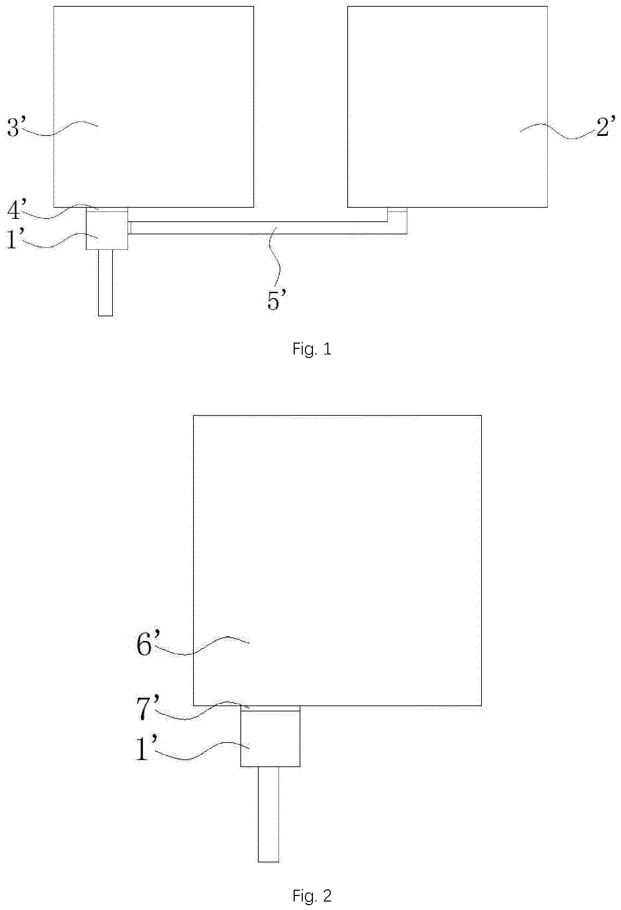

The structure adopting the first mode is shown in Fig. 1, an endoscope body 1' is connected with a cold light source 3' at a joint 4', a processor 2' is connected with the endoscope body 1' through a data connection line 5', a joint is arranged between the processor 2' and the data connection line 5', a joint is also arranged between the data connection line 5' and the endoscope body 1', and more connection joints are provided, thereby reducing the reliability of the endoscope system.

The structure adopting the second mode is shown in Fig. 2, a processor and a cold light source are integrated inside an endoscope host 6', and an endoscope body 1' is connected with the endoscope host 6' at a joint 7', and the second mode improves the stability of the endoscope system compared with the first mode since only one joint is arranged. However, the second mode has the following problems: if wireless power supply is employed, the size of the joint 7' increases due to the large sizes of a power supply coil and a control circuit board, large plugging forces are required during operation, operation is inconvenient, and reliability is low. In order to prevent the endoscope body from falling off from the host, a locking structure that is not easy to operate is generally provided, the connection and detachment of the endoscope body is inconvenient, and the operation is difficult.

### Summary of the Invention

An object of the present invention is to provide an endoscope connection device and an endoscope system aiming at the above problems in the prior art.

The object of the present invention can be achieved by the following technical solution:
a connection locking structure includes a connector I and a connector II, wherein the connector I is connected with the connector II through a magnetic connection assembly.

In the above connection locking structure, the magnetic connection assembly includes a limiting hole formed in the connector I, a limiting post arranged on the connector II, and a magnetic driving member for driving the limiting post to be inserted into or detached from the limiting hole, wherein the magnetic driving member is controlled by a circuit.

In the above connection locking structure, the connector II is provided with a mounting hole opposite to the limiting hole, one end, close to the limiting hole, of the mounting hole is provided with a limiting retaining edge, the limiting retaining edge is provided with a guide hole communicating with the mounting hole, the limiting post is arranged in the guide hole in a penetrating mode, and one end, away from the limiting hole, of the limiting post is provided with a limiting portion.

The limiting portion is located in the mounting hole. The position of movement of the limiting post is limited through the limiting portion, the limiting retaining edge and one end, away from the limiting hole, of the mounting hole to prevent the limiting post from falling off.

In the above connection locking structure, the magnetic driving member includes an elastic member I arranged inside the mounting hole and acting on the limiting post, and an electromagnetic coil controlled by a circuit, wherein the direction of magnetic attraction of the electromagnetic force generated after the electromagnetic coil is energized to the limiting post is opposite to the direction of the elastic force of the elastic member I acting on the limiting post. The limiting portion or the limiting post is a permanent magnet, and the electromagnetic coil and the limiting post attract each other when the electromagnetic coil is energized.

In the above connection locking structure, one end of the elastic member I abuts against one end, away from the limiting hole, of the mounting hole, the other end of the elastic member I abuts against the limiting portion, and the electromagnetic coil is arranged at one end, away from the limiting hole, of the mounting hole. When the connector I and the connector II are connected in place, the limiting post extends from the guide hole and is inserted into the limiting hole under the elastic force of the elastic member I to achieve the purpose of connection locking. When the electromagnetic coil is energized, the electromagnetic coil has magnetic attraction to the limiting portion and the limiting post, the magnetic attraction is greater than the elastic force of the elastic member I, and the limiting post is detached from the limiting hole under the action of the electromagnetic coil.

In the above connection locking structure, one end of the elastic member I abuts against the limiting portion, the other end of the elastic member I abuts against the limiting retaining edge, and the electromagnetic coil is arranged at one end, away from the mounting hole, of the limiting hole. When the connector I and the connector II are not connected, the limiting post is retracted into the mounting hole under the action of the elastic member I; when the connector I and the connector II are connected in place, the electromagnetic coil is powered on to generate an electromagnetic force to attract the limiting post to extend into the limiting hole. In this installation mode, the elastic member I may sleeve the limiting post.

In the above connection locking structure, the connector I is provided with an insertion portion, the connector II is provided with an insertion hole matched with the insertion portion, the mounting hole is formed in a side wall of the insertion hole, and the limiting hole is formed in a side wall of the insertion portion. In order to achieve a better locking effect, the length direction of the limiting post is perpendicular to the length direction of the insertion portion.

In the above connection locking structure, the connector II is provided with an insertion portion, the connector I is provided with an insertion hole matched with the insertion portion, the limiting hole is formed in a side wall of the insertion hole, and the mounting hole is formed in a side wall of the insertion portion.

In the above connection locking structure, the elastic member I is a spring.

A connection device for an endoscope body and an endoscope host includes a connector I, a connector II being in abutting joint with the connector I, and the above connection locking structure, wherein the connection device further includes at least one of the following structures:
a structure I: the connector I is provided with a wire I, the connector II is provided with a wire II opposite to the wire I, and when the connector I is in abutting joint with the connector II, the wire I is connected with the wire II to realize power supply transmission;
a structure II: the connector I is provided with a light conducting member I, the connector II is provided with a light conducting member II, and when the connector I is in abutting joint with the connector II, the light conducting member I is in abutting joint with the light conducting member II to realize the transmission of image signals and control signals; and
a structure III: the connector I is provided with a light conducting member III, the connector II is provided with a light conducting member IV, and when the connector I is in abutting joint with the connector II, the light conducting member III is in abutting joint with the light conducting member IV to realize the transmission of configuration signals.

In the above connection device for the endoscope body and the endoscope host, the connector I is provided with an image signal and control signal optical transmission joint I, one end, close to the connector II, of the image signal and control signal optical transmission joint I is provided with a light guide column I, the light conducting member I is arranged in the image signal and control signal optical transmission joint I in a penetrating mode and is connected with the light guide column I, the connector II is provided with an image signal and control signal optical transmission joint II, one end, close to the connector I, of the image signal and control signal optical transmission joint II is provided with a light guide column II, the light conducting member II is arranged in the image signal and control signal optical transmission joint II in a penetrating mode and is connected with the light guide column II, and one end, away from the connector II, of the image signal and control signal optical transmission joint I and/or one end, away from the connector I, of the image signal and control signal optical transmission joint II is provided with an elastic member II.

In the above connection device for the endoscope body and the endoscope host, the connector I is provided with a sleeve I, the image signal and control signal optical transmission joint I is arranged in the sleeve I, the connector II is provided with a sleeve II, the image signal and control signal optical transmission joint II is arranged in the sleeve II, the elastic member II is arranged at one end, away from the sleeve II, of the sleeve I and/or one end, away from the sleeve I, of the sleeve II, one end, close to the sleeve I, of the sleeve II is provided with a conical surface disposed coaxially with the sleeve II, and the sleeve I abuts against the conical surface. The conical surface is not limited to being disposed on the sleeve II, and the conical surface may also be disposed on the sleeve I, and at this time, the sleeve II abuts against the conical surface.

In the above connection device for the endoscope body and the endoscope host, the light conducting member I is an optical fiber and the light conducting member II is an optical fiber.

In the above connection device for the endoscope body and the endoscope host, the elastic member II is a spring.

In the above connection device for the endoscope body and the endoscope host, the connector I is provided with a power supply connector I, one end, close to the connector II, of the power supply connector I is provided with a metal contact, the wire I is arranged in the power supply connector I in a penetrating mode and is connected with the metal contact, the connector II is provided with a power supply connector II, one end, close to the connector I, of the power supply connector II is provided with metal pins, the wire II is arranged in the power supply connector II in a penetrating mode and is connected with the metal pins, and one end, away from the metal contact, of each metal pin is provided with an elastic member III. The elastic members III can ensure the reliability of the contact between the metal pins and the metal contact, thereby effectively transmitting power and control signals.

In the above connection device for the endoscope body and the endoscope host, each elastic member III is a spring.

In the above connection device for the endoscope body and the endoscope host, the connector I is provided with an air path connecting nozzle, an air path interface I is arranged in the air path connecting nozzle, the connector II is provided with a connection hole matched with the air path connecting nozzle, one end, away from the connector I, of the connection hole is provided with an air path interface II, the air path connecting nozzle extends into the connection hole when the connector I is in abutting joint with the connector II, and the air path interface I communicates with the air path interface II.

In the above connection device for the endoscope body and the endoscope host, a sealing ring is arranged between the air path connecting nozzle and the connection hole. In order to facilitate the mounting of the sealing ring, an annular groove is formed in the inner wall of the connection hole and the sealing ring is mounted in the annular groove.

In the above connection device for the endoscope body and the endoscope host, the connector I is provided with an illumination optical joint I, the connector II is provided with an illumination optical joint II, and the illumination optical joint I is in abutting joint with the illumination optical joint II when the connector I is in abutting joint with the connector II.

An electronic endoscope connector, including the connector I or the connector II described above;
and/or including a signal power transmission part having a first optical signal transmission path, a second optical signal transmission path, and a contact power supply part, wherein the signal power transmission part includes an endoscope body side and a cold light source side;
the first optical signal transmission path includes an electro-optical conversion module at the endoscope body side, the electro-optical conversion module at the endoscope body side converts image electrical signals detected by an endoscope body sensor into image optical signals and transmits the image optical signals to a first optical fiber interface at the cold light source side, and the first optical fiber interface transmits the image optical signals to a photoelectric conversion module of an image processor of an electronic endoscope via optical fibers; the second optical signal transmission path includes a photoelectric conversion module at the endoscope body side, and control signals sent by the image processor of the electronic endoscope are converted by an electro-optical conversion module at the cold light source side, and then transmitted to the photoelectric conversion module at the endoscope body side through a second optical fiber interface; and the contact power supply part includes an isolated power supply at the cold light source side and a contact power supply socket/contact power supply pin connected with the isolated power supply, and further includes a contact power supply pin/contact power supply socket at the endoscope body side, and a power supply unit connected with the contact power supply pin/contact power supply socket at the endoscope body side.

A contact power supply mode is adopted in the present invention, no risk of electromagnetic leakage exists, a small structural size is achieved, and connector miniaturization is facilitated. Two optical signal transmission paths are adopted in the present invention to transmit image information and control information, respectively, to achieve separation of signal transmission from power supply and high interference resistance of the signal.

In one preferred embodiment of the present invention, the connector further includes an illuminating optical interface and a gas supply interface, wherein a cold light source of an electronic endoscope provides illumination light and working gas to an endoscope body through the illumination optical interface and the gas supply interface.

The illumination optical interface, an image information optical interface, the gas supply interface, a power supply interface, and a signal optical interface are arranged on a same connector in the invention, which enables miniaturization of the connector, and all interfaces are connected at one time , which is convenient for insertion.

In order to achieve the above object of the present invention, according to a second aspect of the present invention, the present invention provides an electronic endoscope body, including an endoscope body head end circuit, an operating handle relay circuit, and a data conversion circuit of a light guide part; the endoscope body head end circuit includes a sensor and a parallel-to-serial data conversion unit, wherein the sensor acquires image signals and transmits the image signals to the parallel-to-serial data conversion unit, and the parallel-to-serial data conversion unit converts parallel data into serial data and outputs the serial data to the data conversion circuit of the light guide part; the operating handle relay circuit includes a clock unit and a power supply unit, wherein the clock unit provides a reference clock for the sensor, and the power supply unit at least supplies power to the endoscope body head end circuit and the operating handle relay circuit; and the data conversion circuit of the light guide part includes a serial-to-parallel data conversion unit, a processor, a memory, and a structure at the endoscope body side of the electronic endoscope connector of the invention, wherein the serial-to-parallel data conversion unit receives serial image data, converts the serial image data to parallel image data and transmits the parallel image data to the processor for video format conversion, endoscope body information, video data, and key information are transmitted to a photoelectric conversion module of a first optical signal transmission path, an output signal of a photoelectric conversion module of a second optical signal transmission path is connected with the processor, and the photoelectric conversion module is configured to receive control signals transmitted by an image processor of an electronic endoscope.

The electronic endoscope body of the present invention is connected with the cold light source by using the endoscope connector of the present invention, which can reduce the number of connectors, improve reliability, increase the anti-interference ability of an electronic endoscope system, and reduce the bit error rate of electronic endoscope communication.

In one preferred embodiment of the present invention, the operating handle relay circuit further includes an equalization module and a pre-emphasis module, wherein the parallel-to-serial data conversion unit of the endoscope body head end circuit converts parallel data into serial data and outputs the serial data to the equalization module of the operating handle relay circuit, the equalization module performs equalization processing on the data, then the data after equalization processing is transmitted to the pre-emphasis module, the pre-emphasis module performs emphasis on the data, and then the data after emphasis is transmitted to a serial-to-parallel data conversion unit of a light guide part control circuit.

The eye pattern quality of the digital signal is improved by the processing of the equalization module and the bit error rate is reduced at a data receiving end. The pre-emphasis module increases the amplitude of high-frequency signals, counteracting more attenuation of the high-frequency portion during transmission than low-frequency signals.

In order to achieve the above object of the present invention, according to a third aspect of the present invention, the present invention provides a cold light source of an electronic endoscope, including a light source module and a gas supply module, and further including structures at the endoscope body side and the cold light source side of the electronic endoscope connector of the present invention; a first optical fiber interface of the cold light source of the electronic endoscope receives optical signals output by an electro-optical conversion module at the endoscope body side, and the optical signals are transmitted to an optical fiber connector through optical fibers and are transmitted to an image processor of the electronic endoscope; a connector of the cold light source of the electronic endoscope is connected with a control signal output end of the image processor of the electronic endoscope, electrical control signals are converted into optical control signals through a photoelectric conversion module of a second optical signal transmission path and the optical control signals are output to a photoelectric conversion module at the endoscope body side through a second optical fiber interface; and an isolated power source of the cold light source of the electronic endoscope is connected with a contact power supply socket/contact power supply pin, and the contact power supply socket/contact power supply pin is connected with a contact power supply pin/contact power supply socket at the endoscope body side during operation.

The cold light source of the electronic endoscope of the present invention provides a light source and compressed air to the electronic endoscope while receiving signals from the electronic endoscope and the image processor of the electronic endoscope, providing a forwarding path. The cold light source of the electronic endoscope of the present invention is connected with the endoscope body by using the endoscope connector of the present invention, which can reduce the number of connectors, improve reliability, increase the anti-interference ability of an electronic endoscope system, and reduce the bit error rate of electronic endoscope communication.

In one preferred embodiment of the invention, two transmission channels are formed between the cold light source and the image processor of the electronic endoscope; a light metering value is sent from the image processor to the cold light source by using the first transmission channel, and after the light metering value is received, a cold light source controller controls a current value output by a constant current switching power supply to adjust a luminous flux output of an LED light source; and fault information of the cold light source is sent from the cold light source of the electronic endoscope to the image processor by using the second transmission channel.

The use of the two transmission channels can reduce the response time of light source adjustment, and enables the brightness of a screen to reach a set brightness value faster, not too dark or too bright for a long time.

In order to achieve the above object of the present invention, according to a fourth aspect of the present invention, the present invention provides an electronic endoscope system, including the electronic endoscope body of the present invention, the cold light source of the electronic endoscope of the present invention, and an image processor of an electronic endoscope; the electronic endoscope body is connected with the cold light source of the electronic endoscope through the electronic endoscope connector of the present invention; a photoelectric conversion module of the image processor of the electronic endoscope converts received optical signals into electric signals and transmits the electric signals to a display interface for display.

The electronic endoscope system of the present invention employs two optical signal transmission paths to transmit image information and control information, respectively, to achieve separation of signal transmission from power supply and high interference resistance of the signal.

In one preferred embodiment of the present invention, the image processor of the electronic endoscope includes an FPGA and an ARM, the photoelectric conversion module of the image processor of the electronic endoscope converts the received optical signals into electrical signals and sends the electrical signals into a GTP interface of the FPGA, the FPGA separates video data from information such as key signals, endoscope body information, and the like, signals other than video signals are sent to the ARM through an UART port, the video signals are sent to the ARM after being processed, the ARM sends the received video signals to a display for display, the received key signals enter a corresponding processing interface according to key definition, and the received endoscope body information is sent to the display for display; and the ARM is also connected with an external interface device and receives control instructions sent by the external interface device for corresponding processing.

The reality of the image and the response of the control instructions of the external device are realized.

In another preferred embodiment of the present invention, the electronic endoscope system further includes an optical collimator arranged between an electro-optical conversion module/photoelectric conversion module of the endoscope body and an optical fiber interface of the cold light source of the electronic endoscope. The optical communication interface between the electronic endoscope and the cold light source of the electronic endoscope is provided with an optical collimator to reduce optical transmission loss.

In yet another preferred embodiment of the invention, the cold light source of the electronic endoscope and the image processor of the electronic endoscope are arranged integrally or separately. The design is diverse, and applicability is provided.

An endoscope system, including an endoscope host and an endoscope body, wherein the endoscope host is connected with the endoscope body by the connection device described above.

Compared with the prior art, the present invention has the following advantages:
Through the power supply connector I and the power supply connector II, the wired power supply transmission can be realized, the miniaturization of the connection device can be realized, and the low power consumption of the endoscope body can be realized; the image signals, control signals and configuration signals have strong anti-interference ability at the joint. Through the set magnetic connection structure, the connection and disconnection between the endoscope body and the host can be labor-saving, the connection is more reliable, and good in stability.

### Brief description of the Drawings

Fig. 1 is a structural schematic diagram of a first mode provided in the background.
Fig. 2 is a structural schematic diagram of a second mode provided in the background.
Fig. 3 is a cross-sectional view of a connection locking structure provided in Embodiment 1.
Fig. 4 is a cross-sectional view of a connection locking structure provided in Embodiment 2.
Fig. 5 is a cross-sectional view of a connection locking structure provided in Embodiment 3.
Fig. 6 is a cross-sectional view of a connection locking structure provided in Embodiment 4.
Fig. 7 is a cross-sectional view of a connection device provided in Embodiment 5.
Fig. 8 is a structural schematic diagram of an end surface of a connector I provided in Embodiment 5.
Fig. 9 is a structural schematic diagram of an end surface of a connector II provided in Embodiment 5.
Fig. 10 is a structural schematic diagram of connection between an image signal and control signal optical transmission joint I and an image signal and control signal optical transmission joint II provided in Embodiment 5.
Fig. 11 is a structural schematic diagram of connection between an image signal and control signal optical transmission joint I and an image signal and control signal optical transmission joint II provided in Embodiment 6.
Fig. 12 is a structural schematic diagram of connection between a power supply connector I and a power supply connector II provided in Embodiment 5.
Fig. 13 is a structural schematic diagram of a connection locking structure provided in Embodiment 5.
Fig. 14 is a structural schematic diagram of a connection locking structure provided in Embodiment 7.
Fig. 15 is a cross-sectional view when a limiting hole is connected with a limiting post provided by the present invention.
Fig. 16 is another cross-sectional view when a limiting hole is connected with a limiting post provided by the present invention.
Fig. 17 is another cross-sectional view when a limiting hole is connected with a limiting post provided by the present invention.
Fig. 18 is a schematic cross-sectional diagram of a structure at an endoscope body side of an electronic endoscope connector in one preferred embodiment.
Fig. 19 is a schematic diagram of a circuit structure of an electronic endoscope system in one preferred embodiment.
Fig. 20 is a schematic diagram of a connection structure in Embodiment 11 provided by the present invention.
In the drawings, 1. connector I; 2. connector II; 3. limiting hole; 4. limiting post; 5. mounting hole; 6. limiting retaining edge; 7. limiting portion; 8. elastic member I; 9. electromagnetic coil; 10. insertion portion; 11. insertion hole; 12. light conducting member I; 13. light conducting member II; 14. image signal and control signal optical transmission joint I; 15. light guide column I; 16. image signal and control signal optical transmission joint II; 17. light guide column II; 18. elastic member II; 19. sleeve I; 20. sleeve II; 21. conical surface; 22. power supply connector I; 23. metal contact; 24. power supply connector II; 25. metal pin; 26. elastic member III; 27. air path connecting nozzle; 28. connection hole; 29. sealing ring; 30. illumination optical joint; 31. illumination optical joint II; 32. guide surface; 33. first optical signal transmission path; 34. second optical signal transmission path; 35. contact power supply part; 101. illumination optical interface; 201. image information optical interface; 301. gas supply interface; 401. signal optical interface; and 501. power supply interface.

### Detailed Description of Embodiments

The following are specific embodiments of the invention and further describe the technical solution of the invention in combination with the accompanying drawings, but the invention is not limited to these embodiments.

### Embodiment 1

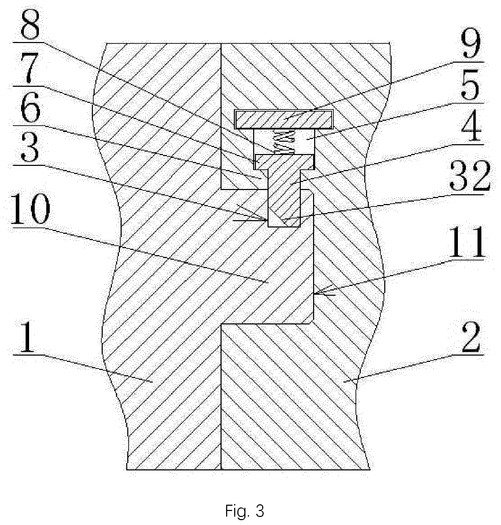

A connection locking structure shown in Fig. 3 is used for locking a connector I 1 and a connector II 2. The connector I 1 is provided with an insertion portion 10, the connector II 2 is provided with an insertion hole 11 matched with the insertion portion 10, and the connection locking structure is arranged between the inner wall of the insertion portion 10 and the side wall of the insertion hole 11.

The connection locking structure includes a connector I 1 and a connector II 2, the connector I 1 is connected with the connector II 2 through a magnetic connection assembly. Specifically, as shown in Fig. 3, the magnetic connection assembly includes a limiting hole 3 formed in the connector I 1, a limiting post 4 arranged on the connector II 2, and a magnetic driving member for driving the limiting post 4 to be inserted into or detached from the limiting hole 3, and the magnetic driving member is controlled by a circuit. The limiting hole 3 extends along the radial direction of the insertion portion 10, the limiting post 4 is arranged coaxially with the limiting hole 3, and the length direction of the limiting post 4 is perpendicular to the insertion direction of the insertion portion 10.

In this embodiment, the inner wall of the insertion hole 11 is provided with a mounting hole 5 opposite to and arranged coaxially with the limiting hole 3, one end, close to the limiting hole 3, of the mounting hole 5 is provided with a limiting retaining edge 6, the limiting retaining edge 6 is provided with a guide hole communicating with the mounting hole 5, the limiting post 4 is arranged in the guide hole in a penetrating mode, and one end, away from the limiting hole 3, of the limiting post 4 is provided with a limiting portion 7 located in the mounting hole 5. The position of movement of the limiting post 4 is limited by the limiting portion 7, the limiting retaining edge 6 and one end, away from the limiting hole 3, of the mounting hole 5, preventing the limiting post 4 from falling off.

As shown in Fig. 3, the magnetic driving member includes an elastic member I 8 arranged inside the mounting hole 5 and acting on the limiting post 4, and an electromagnetic coil 9 controlled by a circuit, one end of the elastic member I 8 abuts against one end, away from the limiting hole 3, of the mounting hole 5, the other end of the elastic member I 8 abuts against the limiting portion 7, and the electromagnetic coil 9 is arranged at one end, away from the limiting hole 3, of the mounting hole 5. The direction of magnetic attraction of the electromagnetic force generated after the electromagnetic coil 9 is energized to the limiting post 4 is opposite to the direction of the elastic force of the elastic member I 8 acting on the limiting post 4.

When the connector I 1 and the connector II 2 are connected in place, the limiting post 4 extends from the guide hole and is inserted into the limiting hole 3 under the elastic force of the elastic member I 8 to achieve the purpose of connection locking. The limiting post 4 is a permanent magnet and when the electromagnetic coil 9 is energized, the electromagnetic coil 9 has magnetic attraction to the limiting portion 7 and the limiting post 4, the magnetic attraction is greater than the elastic force of the elastic member I 8, and the limiting post 4 is detached from the limiting hole 3 under the action of the electromagnetic coil 9. Operation is labor-saving due to being controlled by a circuit.

In order to achieve quick connection, a guide surface 32 is arranged at one end, close to the limiting hole 3, of the limiting post 4. In the process of inserting the insertion portion 10 into the insertion hole 11, the insertion portion 10 abuts against the guide surface 32 and presses the guide surface 32 so as to retract the limiting post 4 into the mounting hole 5. When the limiting post 4 is aligned with the limit hole 3, the limiting post 4 extends into the limiting hole 3 under the action of the elastic member I 8.

The elastic member I 8 in this embodiment is a spring.

Wherein, only one group of connection locking structure or multiple groups of connection locking structures can be arranged between the connector I 1 and the connector II 2. When the multiple groups of connection locking structures are arranged, the groups of connection locking structures are distributed in an annular array, and the limiting posts 4 of the groups of connection locking structures act synchronously.

### Embodiment 2

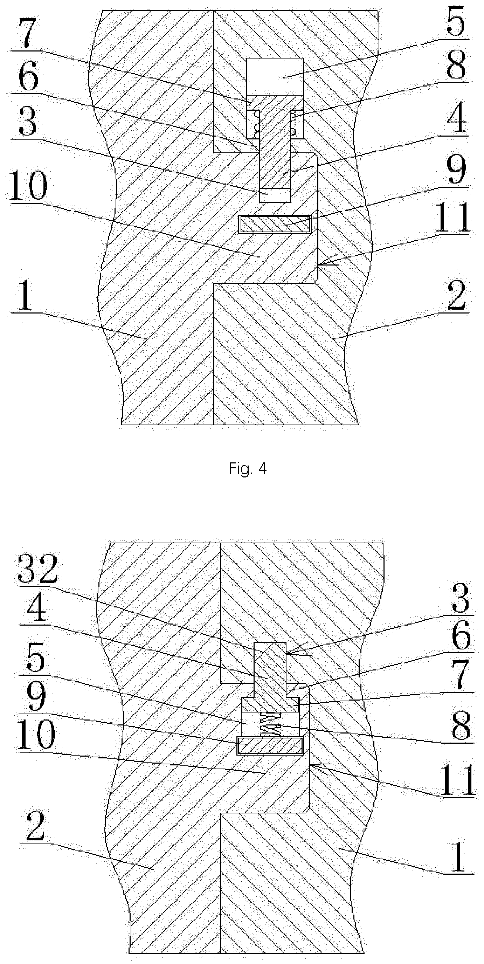

This embodiment provides a connection locking structure for locking a connector I 1 and a connector II 2, the connector I 1 is provided with an insertion portion 10, the connector II 2 is provided with an insertion hole 11 matched with the insertion portion 10, and the connection locking structure is arranged between the inner wall of the insertion portion 10 and the side wall of the insertion hole 11. The structure of the connection locking structure is shown in Fig. 4, including a limiting hole 3 formed in a side wall of the insertion portion 10, a limiting post 4 arranged on a side wall of the insertion hole 11 for insertion into the limiting hole 3, and a magnetic driving member for driving the limiting post 4 to be inserted into or detached from the limiting hole 3, and the magnetic driving member is controlled by a circuit. As shown in Fig. 4, the inner wall of the insertion hole 11 is provided with a mounting hole 5 opposite to and arranged coaxially with the limiting hole 3, one end, close to the limiting hole 3, of the mounting hole 5 is provided with a limiting retaining edge 6, the limiting retaining edge 6 is provided with a guide hole communicating with the mounting hole 5, the limiting post 4 is arranged in the guide hole in a penetrating mode, and one end, away from the limiting hole 3, of the limiting post 4 is provided with a limiting portion 7 located in the mounting hole 5. The position of movement of the limiting post 4 is limited by the limiting portion 7, the limiting retaining edge 6 and one end, away from the limiting hole 3, of the mounting hole 5, preventing the limiting post 4 from falling off.

As shown in Fig. 4, the magnetic driving member includes a spring arranged in the mounting hole 5 and acting on the limiting post 4, and an electromagnetic coil 9 controlled by a circuit, one end of the spring abuts against the limiting portion 7, the other end of the spring abuts against the limiting retaining edge 6, and the electromagnetic coil 9 is arranged at one end, away from the mounting hole 5, of the limiting hole 3, i.e. the electromagnetic coil 9 is arranged in the connector I 1. When the connector I 1 and the connector II 2 are not connected, the limiting post 4 retracts into the mounting hole 5 under the action of the spring; when the connector I 1 and the connector II 2 are connected in place, the electromagnetic coil 9 is energized to generate an electromagnetic force that attracts the limiting post 4 to extend into the limiting hole 3. In this installation mode, the spring can sleeve the limiting post 4.

Wherein, only one group of connection locking structure or multiple groups of connection locking structures can be arranged between the connector I 1 and the connector II 2. When the multiple groups of connection locking structures are arranged, the groups of connection locking structures are distributed in an annular array, and the limiting posts 4 of the groups of connection locking structures act synchronously.

### Embodiment 3

This embodiment provides a connection locking structure for locking a connector I 1 and a connector II 2, the connector II 2 is provided with an insertion portion 10, the connector I 1 is provided with an insertion hole 11 matched with the insertion portion 10, and the connection locking structure is arranged between the inner wall of the insertion portion 10 and the side wall of the insertion hole 11.

As shown in Fig. 5, the connection locking structure includes a limiting hole 3 formed in a side wall of the insertion hole 11, a limiting post 4 arranged on the side wall of the insertion portion 10 for insertion into the limiting hole 3, and a magnetic driving member for driving the limiting post 4 to be inserted into or detached from the limiting hole 3, and the magnetic driving member is controlled by a circuit. The limiting hole 3 extends along the radial direction of the insertion hole 11, the limiting post 4 is arranged coaxially with the limiting hole 3, and the length direction of the limiting post 4 is perpendicular to the insertion direction of the insertion portion 10.

In this embodiment, the inner wall of the insertion portion 10 is provided with a mounting hole 5 opposite to and arranged coaxially with the limiting hole 3, one end, close to the limiting hole 3, of the mounting hole 5 is provided with a limiting retaining edge 6, the limiting retaining edge 6 is provided with a guide hole communicating with the mounting hole 5, the limiting post 4 is arranged in the guide hole in a penetrating mode, and one end, away from the limiting hole 3, of the limiting post 4 is provided with a limiting portion 7 located in the mounting hole 5.

As shown in Fig. 5, the magnetic driving member includes a spring arranged in the mounting hole 5 and acting on the limiting post 4, and an electromagnetic coil 9 controlled by a circuit, one end of the spring abuts against one end, away from the limiting hole 3, of the mounting hole 5 the other end of the spring abuts against the limiting portion 7, and the electromagnetic coil 9 is arranged at one end, away from the limiting hole 3, of the mounting hole 5. The direction of magnetic attraction of the electromagnetic force generated after the electromagnetic coil 9 is energized to the limiting post 4 is opposite to the direction of the elastic force of the elastic member I 8 acting on the limiting post 4.

When the connector I 1 and the connector II 2 are connected in place, the limiting post 4 extends from the guide hole and is inserted into the limiting hole 3 under the elastic force of the elastic member I 8 to achieve the purpose of connection locking. The limiting post 4 is a permanent magnet and when the electromagnetic coil 9 is energized, the electromagnetic coil 9 has magnetic attraction to the limiting portion 7 and the limiting post 4, the magnetic attraction is greater than the elastic force of the elastic member I 8, and the limiting post 4 is detached from the limiting hole 3 under the action of the electromagnetic coil 9.

Wherein, only one group of connection locking structure or multiple groups of connection locking structures can be arranged between the connector I 1 and the connector II 2. When the multiple groups of connection locking structures are arranged, the groups of connection locking structures are distributed in an annular array, and the limiting posts 4 of the groups of connection locking structures act synchronously.

### Embodiment 4

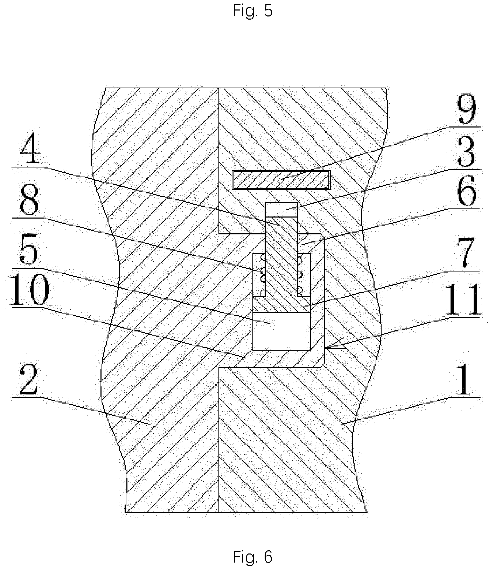

The structural principle of this embodiment is the same as that of Embodiment 3 except that, as shown in Fig. 6, a spring sleeves a limiting post 4 with one end abutting against a limiting portion 7 and the other end abutting against a limiting retaining edge 6, and an electromagnetic coil 9 is arranged at one end, away from a mounting hole 5, of a limiting hole 3, i.e. the electromagnetic coil 9 is arranged inside a connector I 1. When the connector I 1 and a connector II 2 are not connected, the limiting post 4 retracts into the mounting hole 5 under the action of the spring; when the connector I 1 and the connector II 2 are connected in place, the electromagnetic coil 9 is energized to generate an electromagnetic force that attracts the limiting post 4 to extend into the limiting hole 3. In this installation mode, the spring can sleeve the limiting post 4.

### Embodiment 5

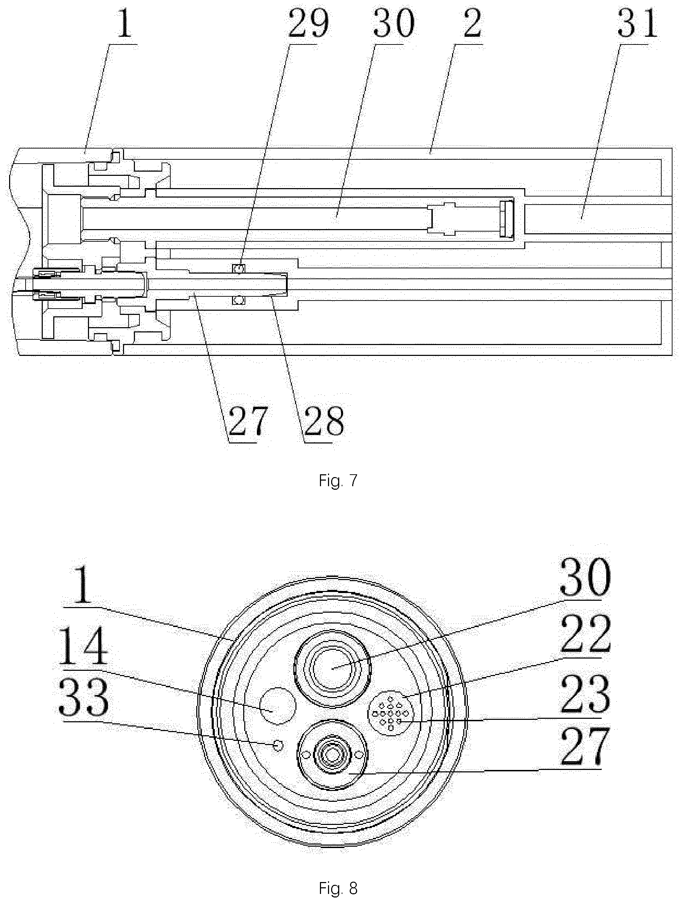

A connection device for an endoscope body and an endoscope host as shown in Figs. 7, 8 and 9 includes a connector I 1, a connector II 2 being in abutting joint with the connector I 1, and a connection locking structure, the connector I 1 is provided with a wire I, the connector II 2 is provided with a wire II opposite to the wire I, and when the connector I 1 is in abutting joint with the connector II 2, the wire I is connected with the wire II to realize power supply transmission; the connector I 1 is provided with a light conducting member I 12, the connector II 2 is provided with a light conducting member II 13, and when the connector I 1 is in abutting joint with the connector II 2, the light conducting member I 12 is in abutting joint with the light conducting member II 13 to realize the transmission of image signals and control signals.

As shown in Figs. 6 and 7, the connector I 1 is provided with a light conducting member III 33, the connector II 2 is provided with a light conducting member IV 34, and when the connector I 1 is in abutting joint with the connector II 2, the light conducting member III 33 is in abutting joint with the light conducting member IV 34 to realize the transmission of configuration signals through infrared signals.

One end of the connector I 1 is connected with the endoscope body and one end of the connector II 2 is connected to the endoscope host.

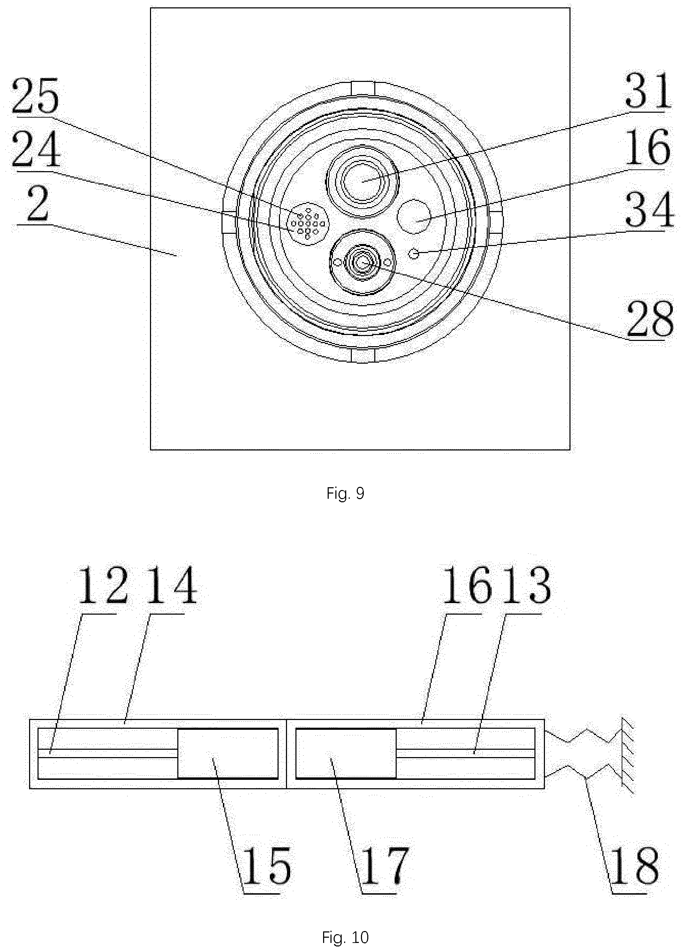

As shown in Fig. 10, the connector I 1 is provided with an image signal and control signal optical transmission joint I 14, one end, close to the connector II 2, of the image signal and control signal optical transmission joint I 14 is provided with a light guide column I 15, the light conducting member I 12 is arranged in the image signal and control signal optical transmission joint I 14 in a penetrating mode and is connected with the light guide column I 15, the connector II 2 is provided with an image signal and control signal optical transmission joint II 16, one end, close to the connector I 1, of the image signal and control signal optical transmission joint II 16 is provided with a light guide column II 17, the light conducting member II 13 is arranged in the image signal and control signal optical transmission joint II 16 in a penetrating mode and is connected with the light guide column II 17, and one end, away from the connector I 1, of the image signal and control signal optical transmission joint II 16 is provided with an elastic member II 18. The elastic member II 18 of this embodiment is a spring.

The light conducting member I 12 is an optical fiber and the light conducting member II 13 is an optical fiber.

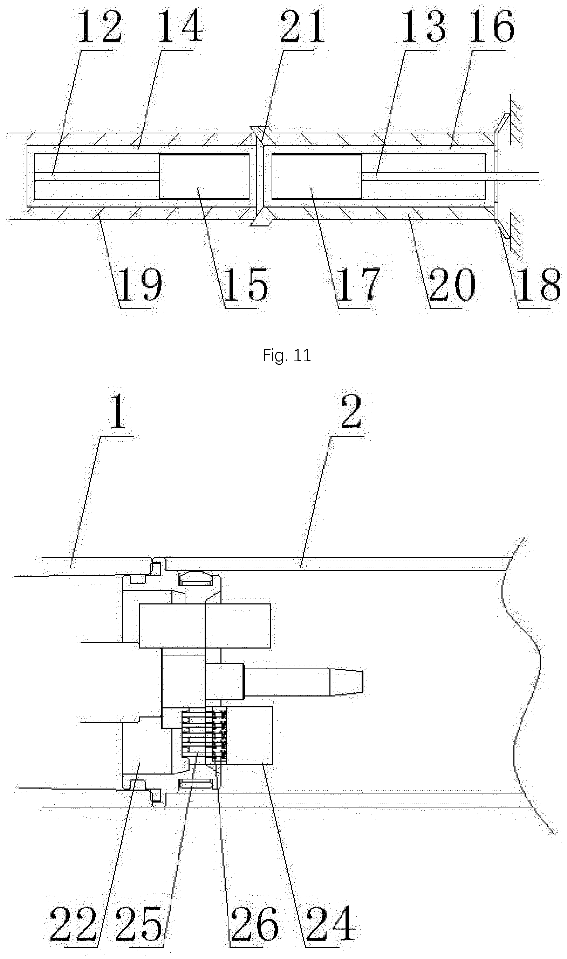

As shown in Figs. 8 and 12, the connector I 1 is provided with a power supply connector I 22, one end, close to the connector II 2, of the power supply connector I 22 is provided with a metal contact 23, and the wire I is arranged in the power supply connector I 22 in a penetrating mode and connected with the metal contact 23; as shown in Figs. 9 and 12, the connector II 2 is provided with a power supply connector II 24, one end, close to the connector I 1, of the power supply connector II 24 is provided with metal pins 25, the wire II is arranged in the power supply connector II 24 in a penetrating mode and connected with the metal pins 25, and one end, away from the metal contact 23, of each metal pin 25 is provided with an elastic member III 26. The elastic members III 26 can ensure the reliability of the contact between the metal pins 25 and the metal contact 23, thereby effectively transmitting power and control signals.

In particular, each elastic member III 26 is a spring.

As shown in Figs. 7 and 8, the connector I 1 is provided with an air path connecting nozzle 27, and an air path interface I is arranged in the air path connecting nozzle 27; as shown in Figs. 7 and 9, the connector II 2 is provided with a connection hole 28 matched with the air path connecting nozzle 27, one end, away from the connector I 1, of the connection hole 28 is provided with an air path interface II, the air path connecting nozzle 27 extends into the connection hole 28 when the connector I 1 is in abutting joint with the connector II 2, and the air path interface I communicates with the air path interface II.

As shown in Fig. 7, a sealing ring 29 is arranged between the air path connecting nozzle 27 and the connection hole 28. In order to facilitate the mounting of the sealing ring 29, an annular groove is formed in the inner wall of the connection hole 28 and the sealing ring 29 is mounted in the annular groove.

As shown in Fig. 7, the connector I 1 is provided with an illumination optical joint I 30, the connector II 2 is provided with an illumination optical joint II 31, and the illumination optical joint I 30 is in abutting joint with the illumination optical joint II 31 when the connector I 1 is in abutting joint with the connector II 2.

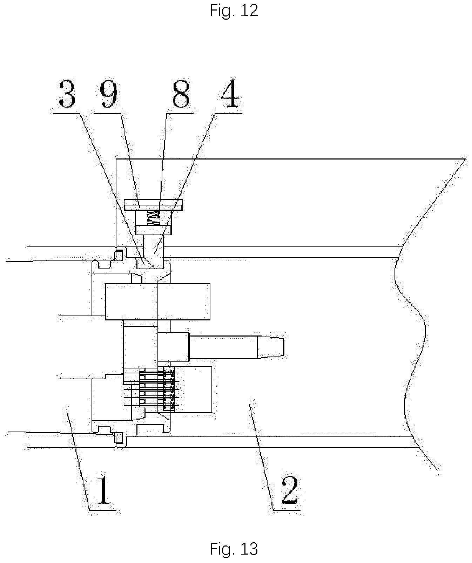

The connector I 1 is provided with an insertion portion 10, the connector II 2 is provided with an insertion hole 11, and the insertion portion 10 is inserted into the insertion hole 11 ; the connection locking structure adopted in this embodiment is shown in Fig. 13, and includes a limiting hole 3 formed in a side wall of the insertion portion 10, a limiting post 4 arranged on a side wall of the insertion hole 11 for insertion into the limiting hole 3, and a magnetic driving member for driving the limiting post 4 to be inserted into or detached from the limiting hole 3, and the magnetic driving member is controlled by a circuit. The limiting hole 3 extends along the radial direction of the insertion portion 10, the limiting post 4 is arranged coaxially with the limiting hole 3, and the length direction of the limiting post 4 is perpendicular to the insertion direction of the insertion portion 10.

The inner wall of the insertion hole 11 is provided with a mounting hole 5 opposite to and arranged coaxially with the limiting hole 3, one end, close to the limiting hole 3, of the mounting hole 5 is provided with a limiting retaining edge 6, the limiting retaining edge 6 is provided with a guide hole communicating with the mounting hole 5, the limiting post 4 is arranged in the guide hole in a penetrating mode, and one end, away from the limiting hole 3, of the limiting post 4 is provided with a limiting portion 7 located in the mounting hole 5.

As shown in Fig. 13, the magnetic driving member includes an elastic member I 8 arranged inside the mounting hole 5 and acting on the limiting post 4, and an electromagnetic coil 9 controlled by a circuit, one end of the elastic member I 8 abuts against one end, away from the limiting hole 3, of the mounting hole 5, the other end of the elastic member I 8 abuts against the limiting portion 7, and the electromagnetic coil 9 is arranged on the connector II 2, facilitating supplying power to the electromagnetic coil 9. The direction of magnetic attraction of the electromagnetic force generated after the electromagnetic coil 9 is energized to the limiting post 4 is opposite to the direction of the elastic force of the elastic member I 8 acting on the limiting post 4.

### Embodiment 6

The structural principle of this embodiment is substantially the same as that of Embodiment 5 except that, as shown in Fig. 11, the connector I 1 is provided with a sleeve I 19, the image signal and control signal optical transmission joint I 14 is arranged in the sleeve I 19, the connector II 2 is provided with a sleeve II 20, the image signal and control signal optical transmission joint II 16 is arranged in the sleeve II 20, the elastic member II 18 is arranged at one end, away from the sleeve I 19, of the sleeve II 20, one end, close to the sleeve I 19, of the sleeve II 20 is provided with a conical surface 21 disposed coaxially with the sleeve II 20, and the sleeve I 19 abuts against the conical surface 21. Wherein the elastic member II 18 is a Belleville spring, and under the action of the Belleville spring, the sleeve I 19 is in close contact with the conical surface 21, ensuring that the axes of the two coincide.

### Embodiment 7

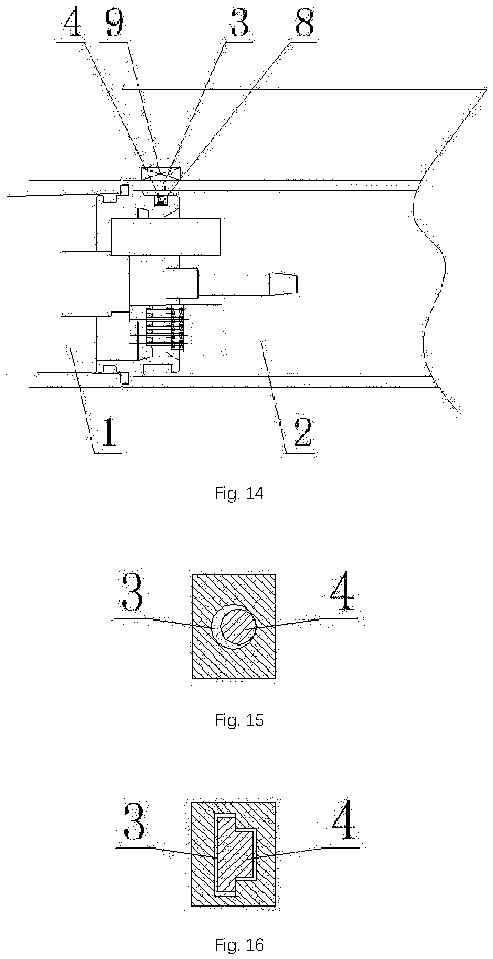

The structural principle of this embodiment is substantially the same as that of Embodiment 5 except that, as shown in Fig. 14, a spring sleeves a limiting post 4 with one end abutting against a limiting portion 7 and the other end abutting against a limiting retaining edge 6, and an electromagnetic coil 9 is arranged inside a connector I 1. When the connector I 1 and a connector II 2 are not connected, the limiting post 4 retracts into the mounting hole 5 under the action of the spring; when the connector I 1 and the connector II 2 are connected in place, the electromagnetic coil 9 is energized to generate an electromagnetic force that attracts the limiting post 4 to extend into the limiting hole 3. In this installation mode, the spring can sleeve the limiting post 4.

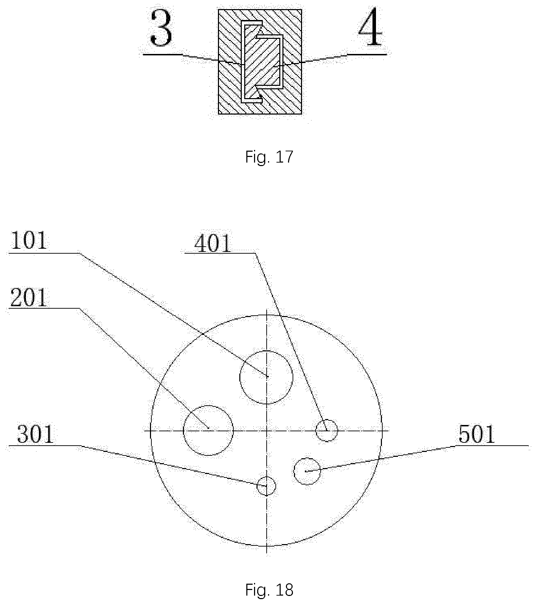

The cross-sectional shape of the limiting hole 3 is the same as the cross-sectional shape of the limiting post 4, the cross-sectional shape of the limiting post 4 is as shown in Figs. 15-17 and may be circular, rectangular or a dovetail mortise and tenon structure. When the limiting post 4 is circular in cross section, as shown in Fig. 15, the limiting post 4 is in line contact with the limiting hole 3; when the limiting post 4 is rectangular in cross section, as shown in Fig. 16, the limiting post 4 is in direct surface contact with the limiting hole 3; when the limiting post 4 is of the dovetail mortise and tenon structure in cross section, as shown in Fig. 17, the limiting post 4 is in inclined contact with the limiting hole 3.

### Embodiment 8

This embodiment provides an endoscope system, including an endoscope host and an endoscope body, wherein the endoscope host is connected with the endoscope body by the connection device in Embodiment 5 or Embodiment 6 or Embodiment 7.

### Embodiment 9

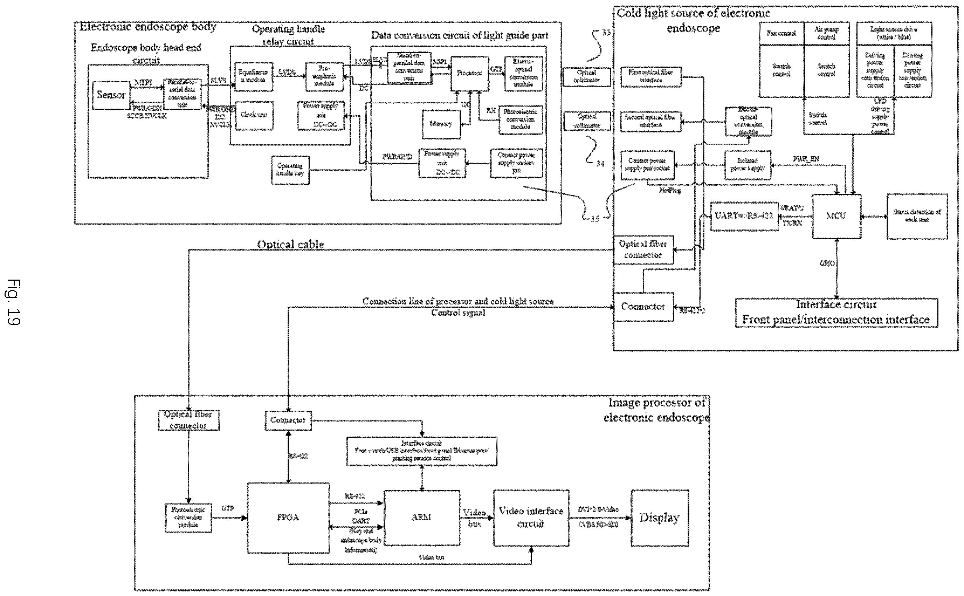

The present invention provides an endoscope system capable of solving the electromagnetic interference problem while facilitating the miniaturization of the connector. As shown in Fig. 19, the present invention discloses an electronic endoscope connector, including the connector I in Embodiment 1; and a signal power transmission part having a first optical signal transmission path 33, a second optical signal transmission path 34, and a contact power supply part 35, wherein the signal power transmission part includes an endoscope body side and a cold light source side.

In another embodiment, only a signal power transmission part having a first optical signal transmission path 33, a second optical signal transmission path 34, and a contact power supply part 35 is included, and the signal power transmission section includes an endoscope body side and a cold light source side.

The first optical signal transmission path includes an electro-optical conversion module at the endoscope body side, the electro-optical conversion module at the endoscope body side converts image electrical signals detected by an endoscope body sensor into image optical signals and transmits the image optical signals to a first optical fiber interface at the cold light source side, and the first optical fiber interface transmits the image optical signals to a photoelectric conversion module of an image processor of an electronic endoscope via optical fibers.

The second optical signal transmission path includes a photoelectric conversion module at the endoscope body side, and control signals sent by the image processor of the electronic endoscope are converted by an electro-optical conversion module at the cold light source side, and then transmitted to the photoelectric conversion module at the endoscope body side through a second optical fiber interface.

The contact power supply part includes an isolated power supply at the cold light source side and a contact power supply socket/contact power supply pin connected with the isolated power supply, and further comprises a contact power supply pin/contact power supply socket at the endoscope body side, and a power supply unit connected with the contact power supply pin/contact power supply socket at the endoscope body side.

A contact power supply mode is adopted in the present invention, no risk of electromagnetic leakage exists, a small structural size is achieved, and connector miniaturization is facilitated. Two optical signal transmission paths are adopted in the present invention to transmit image information and control information, respectively, to achieve separation of signal transmission from power supply and high interference resistance of the signal.

In this embodiment, the connector further includes an illumination optical interface 101 and a gas supply interface 301 , wherein a cold light source of an electronic endoscope provides illumination light and working gas to an endoscope body through the illumination optical interface 101 and the gas supply interface 301. As shown in Fig. 18, the connector (i.e. the connector at the endoscope body side) is provided with the illumination optical interface 101, an image information optical interface 201, the gas supply interface 301, a power supply interface 501, and a signal optical interface 401 as seen from the endoscope body side. All the interfaces are provided on the same connector, which enables miniaturization of the connector, and all the interfaces are connected at one time, which is convenient for insertion.

The present invention also provides an electronic endoscope body, as shown in Fig. 19, comprising an endoscope body head end circuit, an operating handle relay circuit, and a data conversion circuit of a light guide part. Wherein the endoscope body head end circuit includes a sensor and a parallel-to-serial data conversion unit, wherein the sensor acquires image signals and transmits the image signals to the parallel-to-serial data conversion unit, and the parallel-to-serial data conversion unit converts parallel data into serial data and outputs the serial data to the data conversion circuit of the light guide part.

The operating handle relay circuit includees a clock unit and a power supply unit, wherein the clock unit controls the acquisition frequency of the sensor, and the power supply unit at least supplies power to the endoscope body head end circuit and the operating handle relay circuit, and specifically the parallel-to-serial data conversion unit in the endoscope body head end circuit is connected with a serial-to-parallel data conversion unit of the data conversion circuit of the light guide part to enable data transmission (not shown in the drawing). In Fig. 19, another connection is shown, where the endoscope body head end circuit is connected with the data conversion circuit of the light guide part through the operating handle relay circuit.

The data conversion circuit of the light guide part includes a serial-to-parallel data conversion unit, a processor, a memory, and a structure on the endoscope body side of the electronic endoscope connector of the present invention, wherein the serial-to-parallel data conversion unit receives serial image data, converts the serial image data to parallel image data and transmits the parallel image data to the processor for video format conversion, endoscope body information, and video data are transmitted to a photoelectric conversion module of a first optical signal transmission path, an output signal of a photoelectric conversion module of a second optical signal transmission path is connected with the processor, and the photoelectric conversion module is configured to receive control signals transmitted by an image processor of an electronic endoscope.

In this embodiment, the processor may specifically be, but is not limited to, a model XC7K410T-2FFG900I, and may be connected to the serial-to-parallel data conversion unit and the photoelectric conversion module in a general connection mode.

The data conversion circuit of the light guide part is further provided with a power supply unit, which takes power from the contact power supply pin/contact power supply socket at the endoscope body side, and may be arranged integrally with the power supply unit on the operating handle relay circuit (not shown in the drawing) or both may be arranged separately as shown in Fig. 19.

In this embodiment, the sensor, the parallel-to-serial data conversion unit, the clock unit, the power supply unit, the serial-to-parallel data conversion unit, and the memory each adopt a structure commonly used in conventional electronic endoscopes.

The processor performs video format conversion and specifically may, but is not limited to, convert digital image data into transmission data by using existing data converters.

As shown in Fig. 19, in one preferred embodiment of the present invention, due to the small space of a hard part at the head end of the endoscope body, few circuit components can be arranged. The MIPI image signals from the sensor are transmitted as SLVS signals through converting parallel data to serial data. The control, clock signal and power supply of the sensor are provided by the operating handle relay circuit. The baud rate of head-end video signal SLVS exceeds 2 Gbps, and the video signals are transmitted to an operating handle relay board through high-speed cable.

The operating handle relay circuit converts the SLVS signals to LVDS signals, and the operating handle relay circuit further includes an equalization module (an equalizer) and a pre-emphasis module (an emphasizer), wherein the parallel-to-serial data conversion unit of the endoscope body head end circuit converts parallel data into serial data and outputs the serial data to the equalization module of the operating handle relay circuit, the equalization module performs equalization processing on the data, then the data after equalization processing is transmitted to the pre-emphasis module, the pre-emphasis module performs emphasis on the data, and then the data after emphasis is transmitted to a serial-to-parallel data conversion unit of a light guide part control circuit. The purpose of the equalization is to make the eye pattern of digital signals better, so that the bit error rate can be reduced at the data receiving end. In the data stream, the digital signal consisting of 0101 is transmitted. When data of 0101 appears, the frequency of a square wave of the digital signal is equal to the baud rate, but when data of 0011 appears, the frequency of the square wave of the digital signal is only half of the baud rate; due to the bandwidth limitation of the cable transmitting the signal, the higher the frequency, the greater the attenuation of the signal. Therefore, the attenuation amplitude of the high-frequency signal in the data will be greater than that of the low-frequency signal. The purpose of the pre-emphasis is to increase the amplitude of high-frequency signals, counteracting more attenuation of the high-frequency portion during transmission than low-frequency signals. The operating handle relay board sends out the video signal after equalization and pre-emphasis, and the sent video signal is connected to a light guide part control board through a cable.

In this embodiment, the equalization module and the pre-emphasis module are integrally arranged, which may specifically be, but are not limited to, DS25BR100T, and is connected to the parallel-to-serial data conversion unit, and the serial-to-parallel data conversion unit in a general connection mode.

An operating handle is provided with at least one operating handle key, and a signal output end of the operating handle key is connected with a key signal input end of the processor. In one more preferred embodiment of the invention, the operating handle has four keys, and the four key signals are connected to the light guide part control board.

The electronic endoscope body also includes a sensor register and a light guide part memory independently or integrally arranged. The sensor register and the light guide part memory are respectively connected with the processor. Fig. 19 is an integral arrangement structure, and the memory of the data conversion circuit of the light guide part also stores the endoscope body information.

In one more preferred embodiment of the present invention, the specific implementation of the light guide part control board is as follows: the video signal is deserialized and the video format is converted by an FPGA in the light guide part control board, while the key signals, endoscope body information and video data are encoded and sent out to a photoelectric conversion module via a GTP interface of the FPGA. An output signal of an optical receiving module is connected to the FPGA, and the optical receiving module receives control signals such as sensor register configuration information and white balance parameters sent by the image processor of the electronic endoscope. The sensor register is configured bu using the sensor register configuration information received by the FPGA through an I2C bus, and the received white balance parameters are written to the light guide part memory (EEPROM). The EEPROM is also used to store the endoscope body information, such as the endoscope body serial number, production manufacturing information, endoscope body specification parameters, and the like. The power supply of the whole electronic endoscope body is provided by the cold light source of the electronic endoscope through a contact connector.

The connection between the electronic endoscope and the cold light source of the electronic endoscope of the present invention is insulated, and the power supply is provided to the electronic endoscope through the isolated power supply. The optical communication interface between the electronic endoscope and the cold light source of the electronic endoscope is provided with an optical collimation module to reduce optical transmission loss.

With the hardware solution of the present invention, the bit error rate of electronic endoscope communications can be reduced, and in particular, the number of connectors can be reduced and reliability can be improved. At the same time, the anti-interference ability of the electronic endoscope system is greatly improved.

The present invention also provides a cold light source of an electronic endoscope, as shown in Fig. 19, including a light source module and a gas supply module, and further including structures at the endoscope body side and the cold light source side of the electronic endoscope connector of the present invention. A first optical fiber interface of the cold light source of the electronic endoscope receives optical signals output by an electro-optical conversion module at the endoscope body side, and the optical signals are transmitted to an optical fiber connector through optical fibers and are transmitted to an image processor of the electronic endoscope. A connector of the cold light source of the electronic endoscope is connected with a control signal output end of the image processor of the electronic endoscope, electrical control signals are converted into optical control signals through a photoelectric conversion module of a second optical signal transmission path and the optical control signals are output to a photoelectric conversion module at the endoscope body side through a second optical fiber interface. An isolated power source of the cold light source of the electronic endoscope is connected with a contact power supply socket/contact power supply pin, and the contact power supply socket/contact power supply pin is connected with a contact power supply pin/contact power supply socket at the endoscope body side during operation.

The cold light source of the electronic endoscope provides a light source and compressed air for the electronic endoscope, while receiving signals from the electronic endoscope and the image processor of the electronic endoscope, providing a forwarding path.

Specifically, in one preferred embodiment of the present invention, the first optical fiber interface receives encoded optical signals from the electronic endoscope, and the received encoded optical signals are then transmitted to the optical fiber connector through optical fibers. A control signal TX from the image processor of the electronic endoscope is connected to the connector of the cold light source, electrical control signals are converted into optical control signals via a photoelectric conversion module, and the optical control signals are sent out via the second optical fiber interface. The power signal of the electronic endoscope is provided by the cold light source of the electronic endoscope through an isolated power module, and output through the contact power supply socket.

The present invention also provides an electronic endoscope system, as shown in Fig. 19, including the electronic endoscope body of the present invention, the cold light source of the electronic endoscope of the present invention, and an image processor of an electronic endoscope. Wherein, the electronic endoscope body is connected with the cold light source of the electronic endoscope through the electronic endoscope connector of the present invention. A photoelectric conversion module of the image processor of the electronic endoscope converts received optical signals into electric signals and transmits the electric signals to a display interface for display.

An image sensor at the head end of the endoscope body collects images to be sent to a display for display, the transmission distance is far, some even greater than 6m, the transmission rate is high, and the requirements for anti-interference ability are very high. The invention effectively solves the hardware solution of long-distance high interference resistance.

In this embodiment, two transmission channels are formed between the cold light source and the image processor of the electronic endoscope (specifically, but not limited to, an RS-422 transmission channel); a light metering value is sent from the image processor to the cold light source by using the first transmission channel, and after a cold light source controller receives the light metering value, the light metering value is calculated, and the cold light source controller controls a current value output by a constant current switching power supply to adjust a luminous flux output of an LED light source; fault information of the cold light source is sent from the cold light source of the electronic endoscope to the image processor by using the second transmission channel, specifically, connection to the ARM is used for sending the fault information of the cold light source from the cold light source of the electronic endoscope to the image processor, and the information received by the ARM is displayed through a display. Using two channels can reduce the response time of light source adjustment, and enables the brightness of a screen to reach a set brightness value faster, not too dark or too bright for a long time. The communication link between the image processor and the cold light source is shortened through the two channels, so that the cold light source can respond to the dimming response sent by the image processor faster, and shorten the time when the screen is too bright or too dark. Adding an endoscope body in-place detection mechanism can reduce the operation steps of manual power-on or power-off by an operator.

In this embodiment, the light metering value is sent from the image processor to the cold light source, after a cold light source controller receives the light metering value, the light metering value is calculated, and the cold light source controller controls the current value output by the constant current switching power supply to adjust a luminous flux output of the LED light source.

A method for detecting insertion of an endoscope body is proposed in a patent CN109247905A previously applied by this company, and the method can be used in this system. When the cold light source MCU detects insertion signals HotPlug of the electronic endoscope, the MCU enables isolation of power supply signals PWR_EN, the MCU reports endoscope body in-position information to the FPGA, and the FPGA is ready to decode image signals. When the endoscope body is removed from the cold light source of the electronic endoscope, HotPlug is set high, the MCU detects that HotPlug is set high, PWR_EN is set low, and the power supply of the electronic endoscope is turned off.

As shown in Fig. 19, the image processor of the electronic endoscope is used for image signal processing, control signal sending, image display and other functions. The photoelectric conversion module converts the received optical signals into electrical signals and sends the electrical signals to the GTP interface of the FPGA. The FPGA separates the video data from the key signals, endoscope body information and other information. The signals other than the video signals are sent to ARM through UART, and the video signals are sent to the ARM through PCIe after a series of processing. The ARM sends the received video signals plus Ul interface information to the display for display, the received key signals enter the corresponding processing interface according to the key definition, and the received endoscope body information is sent to the display for display. At the same time, the ARM receives control instructions sent by the external interface device for corresponding processing.

In addition to the improvements in the first optical signal transmission path and the second optical signal transmission path involved in the image processor of the electronic endoscope, other functions and structures of the image processor of the electronic endoscope can, but are not limited to, adopt the solution disclosed in CN201721276325.8. And the cold light source of the electronic endoscope and the image processor of the electronic endoscope are arranged integrally or separately.

In this embodiment, the optical communication interface between the electronic endoscope and the cold light source of the electronic endoscope is provided with an optical collimator, reducing optical transmission loss.

### Embodiment 10

The structural principle in this embodiment is substantially the same as that of Embodiment 9 except that the connector I in Embodiment 9 is replaced with the connector II in Embodiment 1.

### Embodiment 11

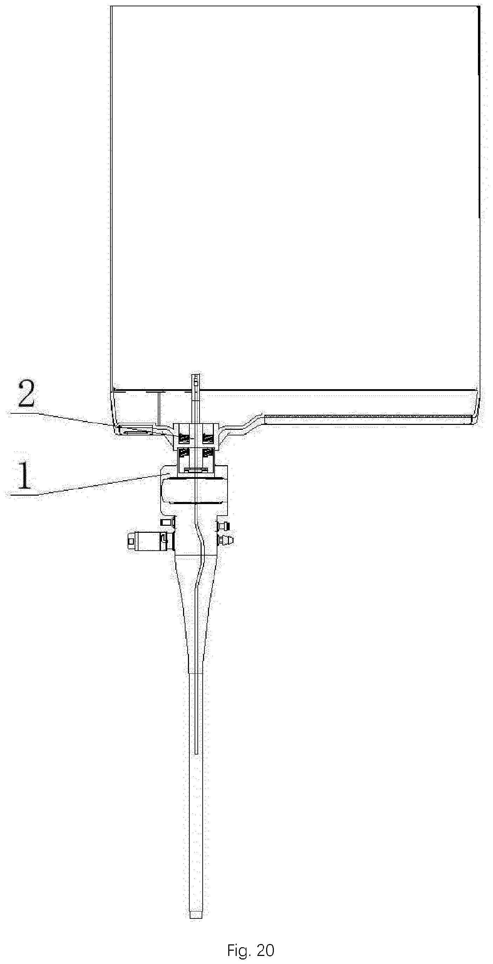

The structural principle in this embodiment is substantially the same as that of Embodiment 1, except that, as shown in Fig. 20, the connector I 1 is a connector of the endoscope body and the connector II 2 is a connector of the endoscope host, and the connector I 1 is in abutting joint with the connector II 2 via a magnetic connection assembly. Wherein the magnetic connection assembly includes an electromagnet I disposed within the connector I 1, and an electromagnet II disposed within the connector II 2, and when electrified, the electromagnet I attracts the electromagnet II to realize the abutting joint between the connector I 1 and the connector II 2.

The connector I 1 is provided with a wire I, the connector II 2 is provided with a wire II opposite to the wire I, and when the connector I 1 is in abutting joint with the connector II 2, the wire I is connected with the wire II to realize power supply transmission; the connector I 1 is provided with a light conducting member I 12, the connector II 2 is provided with a light conducting member II 13, and when the connector I 1 is in abutting joint with the connector II 2, the light conducting member I 12 is in abutting joint with the light conducting member II 13 to realize the transmission of image signals and control signals; and the connector I 1 is provided with a light conducting member III 33, the connector II 2 is provided with a light conducting member IV 34, and when the connector I 1 is in abutting joint with the connector II 2, the light conducting member III 33 is in abutting joint with the light conducting member IV 34 to realize the transmission of configuration signals through infrared signals. The specific embodiments described herein are merely illustrative of the spirit of the present invention. Those skilled in the technical field to which the invention belongs to can make various modifications or supplements to the described specific embodiments or adopt similar methods for replacement without departing from the spirit of the present invention or exceeding the scope defined by the appended claims.

## Claims

1. A connection locking structure, comprising a connector I (1) and a connector II (2), wherein the connector I (1) is connected with the connector II (2) through a magnetic connection assembly.

2. The connection locking structure according to claim 1, wherein the magnetic connection assembly comprises a limiting hole (3) formed in the connector I (1), a limiting post (4) arranged on the connector II (2), and a magnetic driving member for driving the limiting post (4) to be inserted into or detached from the limiting hole (3), and the magnetic driving member is controlled by a circuit.

3. The connection locking structure according to claim 2, wherein the connector II (2) is provided with a mounting hole (5) opposite to the limiting hole (3), one end, close to the limiting hole (3), of the mounting hole (5) is provided with a limiting retaining edge (6), the limiting retaining edge (6) is provided with a guide hole communicating with the mounting hole (5), the limiting post (4) is arranged in the guide hole in a penetrating mode, and one end, away from the limiting hole (3), of the limiting post (4) is provided with a limiting portion (7).

4. The connection locking structure according to claim 3, wherein the magnetic driving member comprises an elastic member I (8) arranged inside the mounting hole (5) and acting on the limiting post (4), and an electromagnetic coil (9) controlled by a circuit, and the direction of magnetic attraction of the electromagnetic force generated after the electromagnetic coil (9) is energized to the limiting post (4) is opposite to the direction of the elastic force of the elastic member I (8) acting on the limiting post (4).

5. The connection locking structure according to claim 4, wherein one end of the elastic member I (8) abuts against one end, away from the limiting hole (3), of the mounting hole (5), the other end of the elastic member I (8) abuts against the limiting portion (7), and the electromagnetic coil (9) is arranged at one end, away from the limiting hole (3), of the mounting hole (5).

6. The connection locking structure according to claim 4, wherein one end of the elastic member I (8) abuts against the limiting portion (7), the other end of the elastic member I (8) abuts against the limiting retaining edge (6), and the electromagnetic coil (9) is arranged at one end, away from the mounting hole (5), of the limiting hole (3).

7. The connection locking structure according to claim 5 or claim 6, wherein the connector I (1) is provided with an insertion portion (10), the connector II (2) is provided with an insertion hole (11) matched with the insertion portion (10), the mounting hole (5) is formed in a side wall of the insertion hole (11), and the limiting hole (3) is formed in a side wall of the insertion portion (10).

8. The connection locking structure according to claim 5 or claim 6, wherein the connector II (2) is provided with an insertion portion (10), the connector I (1) is provided with an insertion hole (11) matched with the insertion portion (10), the limiting hole (3) is formed in a side wall of the insertion hole (11), and the mounting hole (5) is formed in a side wall of the insertion portion (10).

9. A connection device for an endoscope body and an endoscope host, comprising a connector I (1), a connector II (2) being in abutting joint with the connector I (1), and the connection locking structure according to any one of claims 1-8, wherein the connection device further comprises at least one of the following structures:
a structure I: the connector I (1) is provided with a wire I, the connector II (2) is provided with a wire II opposite to the wire I, and when the connector I (1) is in abutting joint with the connector II (2), the wire I is connected with the wire II to realize power supply transmission;
a structure II: the connector I (1) is provided with a light conducting member I (12), the connector II (2) is provided with a light conducting member II (13), and when the connector I (1) is in abutting joint with the connector II (2), the light conducting member I (12) is in abutting joint with the light conducting member II (13) to realize the transmission of image signals and control signals; and
a structure III: the connector I (1) is provided with a light conducting member III (33), the connector II (2) is provided with a light conducting member IV (34), and when the connector I (1) is in abutting joint with the connector II (2), the light conducting member III (33) is in abutting joint with the light conducting member IV (34) to realize the transmission of configuration signals.

10. The connection device for the endoscope body and the endoscope host according to claim 9, wherein the connector I (1) is provided with an image signal and control signal optical transmission joint I (14), one end, close to the connector II (2), of the image signal and control signal optical transmission joint I (14) is provided with a light guide column I (15), the light conducting member I (12) is arranged in the image signal and control signal optical transmission joint I (14) in a penetrating mode and is connected with the light guide column I (15), the connector II (2) is provided with an image signal and control signal optical transmission joint II (16), one end, close to the connector I (1), of the image signal and control signal optical transmission joint II (16) is provided with a light guide column II (17), the light conducting member II (13) is arranged in the image signal and control signal optical transmission joint II (16) in a penetrating mode and is connected with the light guide column II (17), and one end, away from the connector II (2), of the image signal and control signal optical transmission joint I (14) and/or one end, away from the connector I (1), of the image signal and control signal optical transmission joint II (16) is provided with an elastic member II (18).

11. The connection device for the endoscope body and the endoscope host according to claim 10, wherein the connector I (1) is provided with a sleeve I (19), the image signal and control signal optical transmission joint I (14) is arranged in the sleeve I (19), the connector II (2) is provided with a sleeve II (20), the image signal and control signal optical transmission joint II (16) is arranged in the sleeve II (20), the elastic member II (18) is arranged at one end, away from the sleeve II (20), of the sleeve I (19) and/or one end, away from the sleeve I (19), of the sleeve II (20), one end, close to the sleeve I (19), of the sleeve II (20) is provided with a conical surface (21) disposed coaxially with the sleeve II (20), and the sleeve I (19) abuts against the conical surface (21).

12. The connection device for the endoscope body and the endoscope host according to claim 9 or 10 or 11, wherein the connector I (1) is provided with a power supply connector I (22), one end, close to the connector II (2), of the power supply connector I (22) is provided with a metal contact (23), the wire I is arranged in the power supply connector I (22) in a penetrating mode and is connected with the metal contact (23), the connector II (2) is provided with a power supply connector II (24), one end, close to the connector I (1), of the power supply connector II (24) is provided with metal pins (25), the wire II is arranged in the power supply connector II (24) in a penetrating mode and is connected with the metal pins (25), and one end, away from the metal contact (23), of each metal pin (25) is provided with an elastic member III (26).

13. The connection device for the endoscope body and the endoscope host according to claim 9 or 10 or 11, wherein the connector I (1) is provided with an air path connecting nozzle (27), an air path interface I is arranged in the air path connecting nozzle (27), the connector II (2) is provided with a connection hole (28) matched with the air path connecting nozzle (27), one end, away from the connector I (1), of the connection hole (28) is provided with an air path interface II, the air path connecting nozzle (27) extends into the connection hole (28) when the connector I (1) is in abutting joint with the connector II (2), and the air path interface I communicates with the air path interface II.

14. The connection device for the endoscope body and the endoscope host according to claim 13, wherein a sealing ring (29) is arranged between the air path connecting nozzle (27) and the connection hole (28).

15. The connection device for the endoscope body and the endoscope host according to claim 9 or 10 or 11, wherein the connector I (1) is provided with an illumination optical joint I (30), the connector II (2) is provided with an illumination optical joint II (31), and the illumination optical joint I (30) is in abutting joint with the illumination optical joint II (31) when the connector I (1) is in abutting joint with the connector II (2).

16. An electronic endoscope connector, comprising the connector I (1) or the connector II (2) according to any one of claims 1 to 8;
and/or comprising a signal power transmission part having a first optical signal transmission path (33), a second optical signal transmission path (34) and a contact power supply part (35), wherein the signal power transmission part comprises an endoscope body side and a cold light source side;
the first optical signal transmission path comprises an electro-optical conversion module at the endoscope body side, the electro-optical conversion module at the endoscope body side converts image electrical signals detected by an endoscope body sensor into image optical signals and transmits the image optical signals to a first optical fiber interface at the cold light source side, and the first optical fiber interface transmits the image optical signals to a photoelectric conversion module of an image processor of an electronic endoscope via optical fibers;
the second optical signal transmission path comprises a photoelectric conversion module at the endoscope body side, and control signals sent by the image processor of the electronic endoscope are converted by an electro-optical conversion module at the cold light source side, and then transmitted to the photoelectric conversion module at the endoscope body side through a second optical fiber interface; and
the contact power supply part comprises an isolated power supply at the cold light source side and a contact power supply socket/contact power supply pin connected with the isolated power supply, and further comprises a contact power supply pin/contact power supply socket at the endoscope body side, and a power supply unit connected with the contact power supply pin/contact power supply socket at the endoscope body side.

17. The electronic endoscope connector according to claim 16, further comprising an illumination optical interface (101) and a gas supply interface (301), wherein a cold light source of an electronic endoscope provides illumination light and working gas to an endoscope body through the illumination optical interface (101) and the gas supply interface (301).

18. An electronic endoscope body, comprising an endoscope body head end circuit, an operating handle relay circuit, and a data conversion circuit of a light guide part;
the endoscope body head end circuit comprises a sensor and a parallel-to-serial data conversion unit, wherein the sensor acquires image signals and transmits the image signals to the parallel-to-serial data conversion unit, and the parallel-to-serial data conversion unit converts parallel data into serial data and outputs the serial data to the data conversion circuit of the light guide part;
the operating handle relay circuit comprises a clock unit and a power supply unit, wherein the clock unit provides a clock for the sensor, and the power supply unit at least supplies power to the endoscope body head end circuit and the operating handle relay circuit; and
the data conversion circuit of the light guide part comprises a serial-to-parallel data conversion unit, a processor, a memory, and a structure at the endoscope body side of the electronic endoscope connector according to claim 16, wherein the serial-to-parallel data conversion unit receives serial image data, converts the serial image data to parallel image data and transmits the parallel image data to the processor for video format conversion, endoscope body information, video data, and endoscope body key information are transmitted to a photoelectric conversion module of a first optical signal transmission path, an output signal of a photoelectric conversion module of a second optical signal transmission path is connected with the processor, and the photoelectric conversion module is configured to receive control signals transmitted by an image processor of an electronic endoscope.

19. The electronic endoscope body according to claim 18, further comprising at least one operating handle key, wherein a signal output end of the operating handle key is connected with a key signal input end of the processor.

20. The electronic endoscope body according to claim 18, wherein the operating handle relay circuit further comprises an equalization module and a pre-emphasis module, wherein the parallel-to-serial data conversion unit of the endoscope body head end circuit converts parallel data into serial data and outputs the serial data to the equalization module of the operating handle relay circuit, the equalization module performs equalization processing on the data, then the data after equalization processing is transmitted to the pre-emphasis module, the pre-emphasis module performs emphasis on the data, and then the data after emphasis is transmitted to a serial-to-parallel data conversion unit of a light guide part control circuit.

21. A cold light source of an electronic endoscope, comprising a light source module and a gas supply module, and further comprising structures at the endoscope body side and the cold light source side of the electronic endoscope connector according to claim 16;
a first optical fiber interface of the cold light source of the electronic endoscope receives optical signals output by an electro-optical conversion module at the endoscope body side, and the optical signals are transmitted to an optical fiber connector through optical fibers and are transmitted to an image processor of the electronic endoscope;
a connector of the cold light source of the electronic endoscope is connected with a control signal output end of the image processor of the electronic endoscope, electrical control signals are converted into optical control signals through a photoelectric conversion module of a second optical signal transmission path and the optical control signals are output to a photoelectric conversion module at the endoscope body side through a second optical fiber interface; and
an isolated power source of the cold light source of the electronic endoscope is connected with a contact power supply socket/contact power supply pin, and the contact power supply socket/contact power supply pin is connected with a contact power supply pin/contact power supply socket at the endoscope body side during operation.

22. The cold light source of the electronic endoscope according to claim 21, wherein two transmission channels are formed between the cold light source and the image processor of the electronic endoscope;
a light metering value is sent from the image processor to the cold light source by using the first transmission channel, and after the light metering value is received, a cold light source controller controls a current value output by a constant current switching power supply to adjust a luminous flux output of an LED light source; and
fault information of the cold light source is sent from the cold light source of the electronic endoscope to the image processor by using the second transmission channel.

23. An electronic endoscope system, comprising the electronic endoscope body according to one of claims 18-20, the cold light source of the electronic endoscope according to one of claims 21-22, and an image processor of an electronic endoscope; wherein
the electronic endoscope body is connected with the cold light source of the electronic endoscope through the electronic endoscope connector according to claim 16 or 17; and
a photoelectric conversion module of the image processor of the electronic endoscope converts received optical signals into electric signals and transmits the electric signals to a display interface for display.

24. The electronic endoscope system according to claim 23, wherein the image processor of the electronic endoscope comprises an FPGA and an ARM, the photoelectric conversion module of the image processor of the electronic endoscope converts the received optical signals into electrical signals and sends the electrical signals into a GTP interface of the FPGA, the FPGA separates video data from information such as key signals, endoscope body information, and the like, signals other than video signals are sent to the ARM through an UART port, the video signals are sent to the ARM after being processed, the ARM sends the received video signals to a display for display, the received key signals enter a corresponding processing interface according to key definition, and the received endoscope body information is sent to the display for display; and
the ARM is also connected with an external interface device and receives control instructions sent by the external interface device for corresponding processing.

25. The electronic endoscope system according to claim 23, further comprising an optical collimator arranged between an electro-optical conversion module/photoelectric conversion module of the endoscope body and an optical fiber interface of the cold light source of the electronic endoscope.

26. The electronic endoscope system according to claim 23, wherein the cold light source of the electronic endoscope and the image processor of the electronic endoscope are arranged integrally or separately.

27. An endoscope system, comprising an endoscope host and an endoscope body, wherein the endoscope host is connected with the endoscope body by the connection device according to any one of claims 8-15.
